# EUROPÄISCHE PATENTANMELDUNG

(11) **EP 3 892 991 A1**
(43) Veröffentlichungstag der Anmeldung: **13.10.2021**
(21) Anmeldenummer: 21020194.3
(22) Anmeldetag: 09.04.2021
(51) Int. Cl.: G01N 27/622, G01N 33/00, G01N 30/86, A61B 5/08, H01J 49/00, G01N 33/497

(54) **MULTIMARKERMONITOR ZUR ANALYSE FLÜCHTIGER ORGANISCHER VERBINDUNGEN**

(30) Priorität: 09.04.2020 DE 202020001482 U; 03.06.2020 DE 202020002446 U
(71) Anmelder: GRAUPNER medical solutions GmbH, 09468 Geyer (DE)
(72) Erfinder: Becher, Gunther, 16321 Bernau (DE); Graupner, Rolf, 09353 Oberlungwitz (DE); Viertel, Beate, 09126 Chemnitz (DE)
(74) Vertreter: Steiniger, Carmen

(57) **Zusammenfassung**

Die Erfindung betrifft eine Vorrichtung, beinhaltend ein Ionenmobilitätsspektrometer und eine Mikrokapillarsäule bzw. Gaschromatographiesäule, zur medizinischen Diagnostik und Analytik mittels der Analyse von flüchtigen organischen Verbindungen (VOC), insbesondere volatilen Biomarkern, unter anderem im Headspace von Bakterienkulturen oder über biologischen Proben bzw. in der Ausatemluft oder der Haut. Anwendungsgebiete sind die Medizin, Umweltmedizin, Gesundheitsschutz und die Biotechnologie, insbesondere die medizinische Diagnostik.

## Beschreibung

Die Erfindung betrifft eine Vorrichtung zur medizinischen Diagnostik und Analytik mittels der Analyse von flüchtigen organischen Verbindungen (VOC), insbesondere volatilen Biomarkern, unter anderem im Headspace von Bakterienkulturen oder über biologischen Proben bzw. in der Ausatemluft oder der Haut. Anwendungsgebiete sind die Medizin, Umweltmedizin, Gesundheitsschutz und die Biotechnologie, insbesondere die medizinische Diagnostik.

Es ist bekannt, dass in der Ausatemluft von Tier und Mensch mehrere 100 verschiedene Substanzen nachgewiesen werden können. Auch im Headspace über biologischen Proben sind zahlreiche Stoffe vorhanden. Für die Erkennung von ungewöhnlichen Substanzen in der Umgebung gibt es ebenfalls Verfahren, die zum Beispiel für eine Raumluftüberwachung genutzt werden können. Es sind verschiedene Vorrichtungen bekannt, mit denen solche Nachweise durchgeführt werden können, u. a. G. Becher et al DE 102011106717 A1.

Der Erfindung liegt die Aufgabe zugrunde, eine Vorrichtung zu entwickeln, mit der flüchtige organische Verbindungen schnell und sicher analysiert werden können. Sie soll einfach zu bedienen und in breiten Bereichen anwendbar sein. In der Medizin soll eine sichere Erkennung von bakteriellem Wachstum oder bakteriellem Befall ermöglicht werden.
Maßgeblicher Vorteil der Vorrichtung besteht darin, dass flüchtige Substanzen auch unabhängig von der Kenntnis ihrer chemischen Entität einzeln oder in gemeinsamer Betrachtung verschiedener Stoffe einer bestimmten Diagnose, Krankheit, Stoffwechselveränderung, Ernährungsweise zugeordnet werden können. Auch ohne genaue Kenntnis der der jeweiligen chemischen Struktur kann dann auf die zugrundeliegende Stoffwechselleistung rückgeschlossen werden. Bei vorhandener Stichprobe mit Objekten mit den gesuchten Eigenschaften (Lernstichprobe) können beliebige weitere Proben auf das Vorhandensein dieser Eigenschaft untersucht werden.

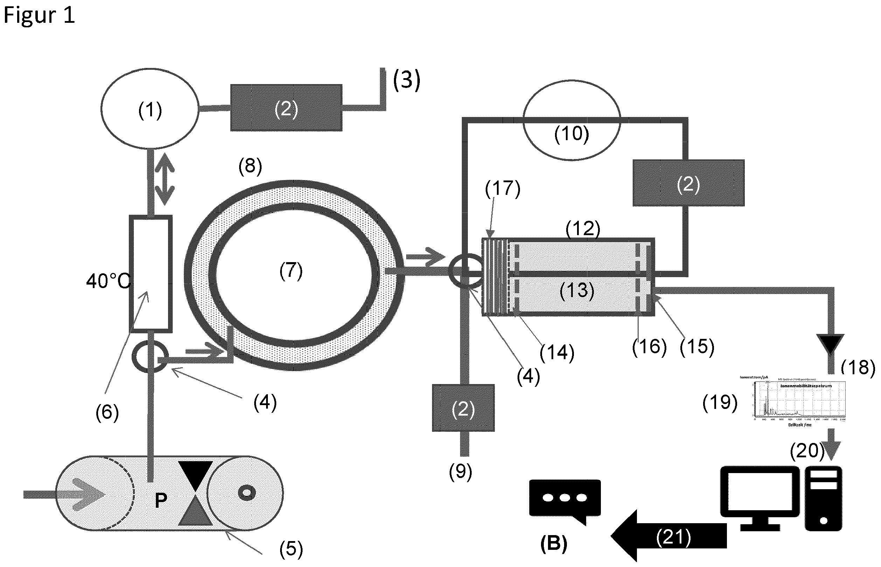

Die Aufgabe wird mit der Vorrichtung gemäß Anspruch 1 gelöst, sie ist in Figur 1 schematisch dargestellt und hat folgende Bestandteile:
(13) Messgerät mit IMS
(17) lonisationsquelle, e.g. Strahlungsquelle unter 100 MBq (unterhalb der Freigrenze), UV Lampe
(7) Mikrokapillarsäule bzw. Gaschromatographiesäule
(2) Luftfilter integriert, zur Reinigung der Systemgase
(6) - (26) Messkreislauf mit Luft (keine externen Reinstgase erforderlich) (20) Eingebauter PC mit Datenbank und Speichermedium und Analysesoftware oder Rechner mit Windows 7 professional (und aufwärts), SSD ab 150 GB
(21) Software "Airinterpreter", auch auf externem Rechner oder im Netzwerk installierfähig, Kommunikationsschnittstellen (Touchscreen, Tastatur, Mouse, Fernbedienung etc.) Steuerung über Netzwerk möglich (z. B. Anbindung an Praxissysteme oder per Teamviewer)
   Anschlüsse: USB, LAN, wLAN, Bluetooth,

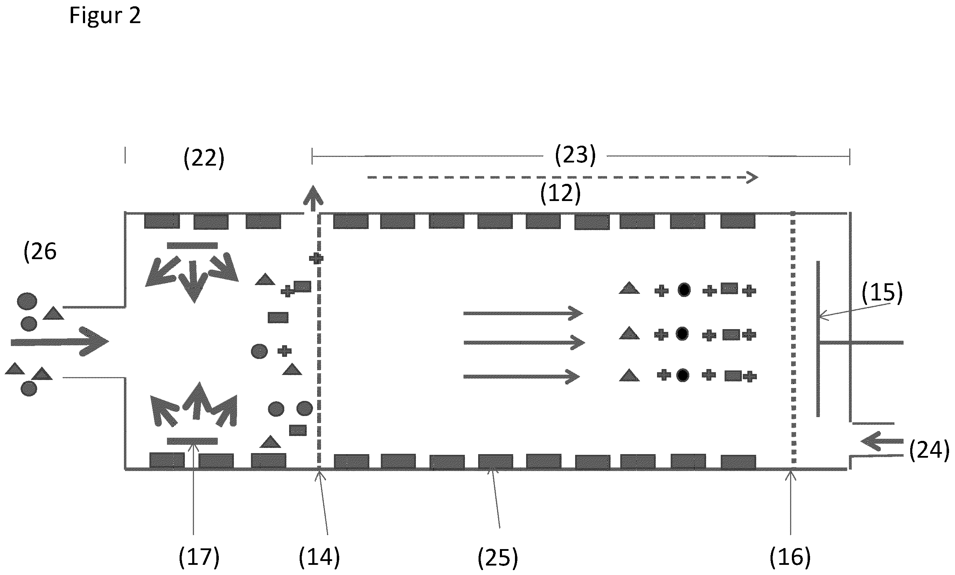

Das Prinzipschema der Messzelle ist in Figur 2 dargestellt.

Die Probe wird mittels **lonen-Mobilitäts-Spektrometer** (IMS) analysiert. Sie wird ionisiert, die Ionen werden bei Umgebungsdruck von einem elektrischen Feld durch Gas/Luft "gezogen", wobei i.d.R. der Gasstrom dem Ionenfluss entgegenwirkt Durch Kollisionen mit den Gasmolekülen werden die Ionen gebremst, wobei diese "Reibungskraft" bei großen Molekülen stärker ist als bei kleinen Molekülen. Deshalb bewegen sich kleine Moleküle in der Regel mit einer höheren Geschwindigkeit im Gas. Entscheidend ist, dass die Ionen Energie im elektrischen Feld zwischen zwei Stößen aufnehmen und bei einem Stoß wieder abgeben. Da dies sehr schnell geschieht, erreichen die Ionen eine für sie charakteristische mittlere Geschwindigkeit. Der Drift im elektrischen Feld wird als Driftgeschwindigkeit bezeichnet. Da diese Driftgeschwindigkeit vor allem wegen der Molekülgröße, aber auch wegen anderer physikalischer Parameter (Polarisierbarkeit) für verschiedene Ionen der Analyt-Moleküle unterschiedlich ist, können diese voneinander unterschieden werden.

Das Ergebnis einer Messung ist dann der Befund für eine Einzelprobe nach Zuordnung seiner Messung zu der vorher erstellten Datenbank, welche (Vergleichs-) Lernstichproben enthält.

Die Erfindung soll nachfolgend durch Beispiele näher erläutert werden.

Technische Daten Multimarkermonitor: Stromversorgung:
- Netzteil extern für 110 - 230 Volt
- Stromversorgung Gerät: 19 Volt bis 21 Volt
- Spritzwassergeschützt
- Umgebungstemperatur: 0°C bis 35 °C,
- vor Inbetriebnahme Temperatur mit Umgebung angleichen

Erkennung von spezifischen Clustern von volatilen Biomarkern in der Luft über Bakterienkulturen, Haut, Hautläsionen (Wunden) bzw. in der Ausatemluft In Studien wurden z. B. spezifische Kombinationen von Biomarkern (Clustern) für unter anderem folgende Konstellationen bzw. Erkrankungen nachgewiesen:
- Mycobacterium avium paratuberculosis (MAP) und
- MRSA versus MSSA im Vergleich zu ubiquitären Bakterien
Weiterhin:
- E. Coli
- Enterobacter
- Klebsiella
   Weiterhin
- Influenca
- Andere Viren
- Andere virale Erkrankungen
   Weiterhin
- Stoffwechselmarker für Diabetes
- Stoffwechselmarker für Fettstoffwechsel
- Spezifische Volatile Stoffe (VOC) in Korrelation zu anderen Erkrankungen (z. B. M. Parkinson, M. Crohn, chronische Herzinsuffizienz

Mit dem Multimarkermonitor werden erfindungsgemäß keine Bakterien oder Viren an sich gemessen, sondern lediglich Volatile Stoffe (VOC), die bei einer floriden Infektion und den durch diese induzierten Stoffwechselvorgänge im Organismus entstehen und freigesetzt werden. Ein Signal entsteht also nur bei aktiver Infektion.

### Durchführung der Messung

Eine gasförmige Probe von 2 - 3 ml wird aktiv eingesaugt und durch eine Trennsäule gepumpt. Durch eine innen liegende Probenschleife wird die Probe exakt über das vorgegebene Volumen der Probenschleife dosiert bzw. volumetrisch bestimmt. Die gasförmige Probe wird dann mit einer Pumpe mit in einem Filter gereinigter Luft durch die Multkapillarsäule oder Gaschromatographiesäule gepumpt. Entsprechend der Wechselwirkungen der Moleküle erfolgt hier eine Trennung der Substanzen nach Molekulargewicht, Wechselwirkung mit der Auskleidung der Säule in Abhängigkeit von der eingestellten Temperatur Danach erfolgt eine Ionisierung der in dem Gas enthaltenen Substanzen. Die Ionen werden entsprechend ihrem Gewicht und der Ladung im elektrischen Feld, dem eigentlichen Sensor, getrennt und detektiert.

### Die Probenahme:

Über eine eingebaute Pumpe (1) wird eine Probe standardisiert eingesaugt und die interne Probenschleife damit gefüllt.
Absaugung einer in-vitro Luftprobe, Kulturflasche, Probenbehälter, Sammelbeutel.... Absaugung einer in-vivo Probe: aus Nase, aus Ausatemluft, über Wundexsudat

Die Probennahme erfolgt wahlweise über ein kommerzielles Abnahmebesteck oder speziell entwickelte Probenahmesysteme. Die Probenahme kann über einen Bakterienfilter oder andere Partikel zurückhaltende Filter erfolgen. Eine Kontamination des Gerätes oder Gefährdung des Personals wird somit ausgeschlossen (keine Infektionsgefahr).

Der Multimarkermonitor hat gegenüber ähnlichen Vorrichtungen oder Verfahren den entscheidenden Vorteil, dass er keiner externen Probenvorbereitung bzw. Probenaufbereitung bedarf, keine Zufuhr von Hilfsmedien braucht und somit breit einsetzbar ist. Das Ergebnis kann schon innerhalb weniger Minuten vorliegen.

### Bakterielle Infektionen

Der Multimarkermonitor soll im direkten Vergleich die traditionellen bakteriologischen Methoden der Bakterienkultur bzw. Anzüchtung der Keime ersetzen, beschleunigen und zusätzlich für das Personal sicherer machen. Bei traditioneller Flüssigkultur ist ein Ergebnis frühestens nach 1 - 2 Stunden (traditionell 24 h) möglich. Die langsam wachsenden Keime (z. B. MAP) werden mit dem Multimarkermonitor nach 1 Woche erkannt, bei der traditionellen Kultur dauert es 12 Wochen!
Auch bei Tuberkulose dauert die Kultur üblicherweise 12 Wochen, mit dem Multimarkermonitor wird ein Ergebnis schon nach einer Woche erwartet. Andere Methoden, wie PCR-Analyse, Enzymimmuno-Assays sind kostenintensiv, an die Laborumgebung gebunden und bedürfen auch einer Zeit bis zum Ergebnis von 2 bis 24 Stunden.

Die breiten Einsatzmöglichkeiten der Vorrichtung sind konkret
- Es erkennt bakteriellen Befall am Geruch der Ausatemluft/Nasenluft
- Es erkennt wachsende Bakterienkulturen im Labor aus der Luft über der Kultur bzw. Luft in der Kulturflasche oder aus Sekreten
- durch "Anlernen" mit vorhandenen Proben können in kürzester Zeit neue Krankheiten oder Bakterien differenziert werden ("Selbstlernendes System")
- Die Auswertesoftware gibt das Ergebnis auch als statistische Maßzahl aus.
- Die Auswertung kann jederzeit mit den Originaldaten wiederholt werden.
- Es ist kosten- und aufwandneutral möglich, verschiedene Fragestellungen mit einer Probe zu bearbeiten. Das ist jederzeit auch nachträglich möglich, wenn eine entsprechende Datenbank für die Fragestellung verfügbar ist oder erstellt werden kann.
- Originaldaten (Spektren) sind unverändert gespeichert und können jederzeit nachträglich verglichen und ausgewertet werden.

### Legende zu den Figuren

Figur 1: Prinzipschema des Multimarkermonitor
Figur 2: Prinzipschema der Messzelle

### Bezugszeichen

(P) Probe
(1) Pumpe
(2) Filter
(3) Auslass
(4) Dreiwegeventil
(5) Atemrohr, wahlweise mit Flussmessung
(6) Trap bzw. Probeschleife
(7) Mikrokapillarsäule bzw. Gaschromatographiesäule
(8) Temperierung 40 - 60 °C
(9) Gasauslass/Druckregulation
(10) IMS Kreislaufpumpe
(13) IMS Sensor mit
   (14) Einlassgitter
   (15) Detektor
   (16) Abschirmgitter
   (17) Ionisationsquelle
   (12) Elektrisches Feld
   (22) Reaktionsraum
   (23) Driftraum
   (24) Driftgas
   (25) Driftringe
   (26) Probeneinlass
(18) Verstärker
(19) Spektrogramme
(20) Eingebauter PC mit Datenbank und Speichermedium und Analysesoftware oder Rechner mit Windows 7 professional (und aufwärts), SSD ab 150 GB
(21) Zuordnung der Messungen zur Datenbank oder Software "Airinterpreter", (B) Befund

## Patentansprüche

1. Multimarkermonitor (Infektmonitor) zur schnellen und sicheren Analyse flüchtiger organischer Verbindungen gemäß Figur 1, **gekennzeichnet durch** folgende Merkmale:
• (13) Messgerät mit IMS
• (17) Ionisationsquelle (z. B. Strahlungsquelle unter 100 MBq (unterhalb der Freigrenze, keine Strahlenschutzgenehmigung erforderlich) oder UV Lampe
• (7) Mikrokapillarsäule bzw. Gaschromatographiesäule
• (2) Luftfilter integriert, zur Reinigung der Systemgase und der Abluft
• (6) - (26) Messkreislauf mit Luft (keine externen Reinstgase erforderlich)
• (20) Eingebauter PC mit Datenbank und Speichermedium und Analysesoftware oder Rechner mit Windows 7 professional (und aufwärts), SSD ab 150 GB
• (21) Zuordnung der Messungen zur Datenbank oder Software "Airinterpreter", der auch auf externem Rechner oder im Netzwerk installierfähig ist,
• Kommunikationsschnittstellen (Touchscreen, Tastatur, Mouse, Fernbedienung etc.)
• Steuerung über Netzwerk möglich (z.B. Anbindung an Praxissysteme oder per Teamviewer)
• Anschlüsse: USB, LAN, wLAN Bluetooth,

2. Multimarkermonitor gemäß Anspruch 1 zur Bestimmung flüchtiger Substanzen unabhängig von der Kenntnis ihrer chemischen Entität einzeln oder in gemeinsamer Betrachtung verschiedener Stoffe einer bestimmten Diagnose, Krankheit, Stoffwechselveränderung, Ernährungsweise.

3. Multimarkermonitor gemäß Anspruch 1 zur sicheren Bestimmung von bakteriellem Wachstum oder bakteriellem Befall oder anderen Infektionen, wie beispielsweise viral bedingte Erkrankungen.

4. Multimarkermonitor gemäß Anspruch 1, **dadurch gekennzeichnet, dass** bei vorhandener Lernstich- /Vergleichsprobe von Objekten mit den gesuchten Eigenschaften andere Erkrankungen, Stoffwechselbesonderheiten, wie beispielsweise Diabetes, Lebererkrankungen, Störungen des Magen-Darm-Traktes, Neurologische Erkrankungen und andere erkannt werden können.

5. Multimarkermonitor gemäß Anspruch 1, **dadurch gekennzeichnet, dass** bei vorhandener Lernstich- /Vergleichsprobe von Objekten mit den gesuchten Eigenschaften beliebige weitere Proben auf das Vorhandensein dieser Eigenschaft untersucht werden können.

6. Multimarkermonitor gemäß Anspruch 1, **dadurch gekennzeichnet, dass** bei vorhandener Datenbank mit einer oder verschiedenen Vergleichsstichproben und mit einer Zusatz-Software zum Airinterpreter (AirClient) eine Ad-Hoc Aussage zu jeder aktuellen Messung ermöglicht wird.
